(19) **Europäisches Patentamt**
**European Patent Office**
**Office européen des brevets**

(11) **EP 3 158 927 B1**

(12) **EUROPEAN PATENT SPECIFICATION**

(45) Date of publication and mention
of the grant of the patent:
**30.06.2021 Bulletin 2021/26**

(51) Int Cl.:
***A61B 5/021*** *(2006.01)*    ***A61B 5/0205*** *(2006.01)*
***A61B 5/08*** *(2006.01)*

(21) Application number: **16193279.3**

(22) Date of filing: **11.10.2016**

(54) **PULSE WAVE ANALYZER**

IMPULSWELLENANALYSATOR

ANALYSEUR D'ONDES DE POULS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.10.2015 JP 2015205536**

(43) Date of publication of application:
**26.04.2017 Bulletin 2017/17**

(73) Proprietor: **Nihon Kohden Corporation
Shinjuku-ku
Tokyo (JP)**

(72) Inventors:
• **Tanishima, Masami
Tokyo (JP)**
• **Sakai, Tomoyuki
Tokyo (JP)**

(74) Representative: **Hoffmann Eitle
Patent- und Rechtsanwälte PartmbB
Arabellastraße 30
81925 München (DE)**

(56) References cited:
**EP-A1- 2 263 528       US-A1- 2009 048 527
US-A1- 2009 156 945**

**Description**

BACKGROUND

The presently disclosed subject matter relates to an apparatus for analyzing a pulse wave which is acquired from a subject.

The respiratory variation of the arterial pressure may be an important index for knowing the circulatory dynamics in the subject. In the case where the circulatory blood volume in the subject is not sufficient, for example, the value of the respiratory variation of the arterial pressure is large. In the case where the circulatory blood volume in the subject is sufficient, by contrast, the value of the respiratory variation of the arterial pressure is small.

JP-T-2011-511686 discloses an apparatus for measuring the pulse pressure variation (respiratory variation) in the respiratory cycle.

US 2009/156945 A1 describes a catheter used to measure blood pressure, a mechanical ventilator used to assist patient in breathing, and a monitoring device used to continuously monitor various parameters associated with the patient, such as the respiratory variation of the pulse pressure. The heart rate may be determined using an electrocardiogram.

US 2009/048527 A1 describes the comparisons of one or more characteristics of a cardiac cycle. Predetermined thresholds are used in the comparisons.

EP 2 263 528 A1 describes a method for determining physiologic parameters of a patient.

In the case where the respiration of the subject is assisted by using an artificial respirator, it is contemplated that the acquisition of the circulatory dynamics in the subject is effective for determining whether the respiration assistance using the artificial respirator is adequately performed or not.

Therefore, the inventors studied a method for acquiring the circulatory dynamics in the subject in whom the respiration is assisted by an artificial respirator, based on the respiratory variation. However, it has been often that, in the case where the respiratory variation is used as an index for knowing the circulatory dynamics in the subject, the accuracy of the respiratory variation which is calculated by using a related-art apparatus is low.

SUMMARY

The presently disclosed subject matter may provide a pulse wave analyzer in which the accuracy of the calculation of the respiratory variation that is an index for knowing the circulatory dynamics in the subject can be improved.

This object is accomplished by the subject-matter of the independent claim . The dependent claims concern particular embodiments.

The pulse wave analyzer according to the invention comprises: a pulse wave acquirer which is configured to acquire a pulse wave of a subject; an electrocardiogram acquirer which is configured to acquire an electrocardiogram of the subject; a respiration information acquirer which is configured to acquire respiration information of the subject; and an analyzing section which includes: a setting section which is configured to set a selection criterion based on the pulse wave or the electrocardiogram, and the respiration information; and a selecting section which is configured to select unit pulse waves in said pulse wave based on the selection criterion, wherein the analyzing section is configured to calculate a respiratory variation from the pulse wave which is selected by the selection section.

BRIEF DESCRIPTION OF THE DRAWINGS

Fig. 1 is a diagram illustrating a pulse wave analyzer of an embodiment of the presently disclosed subject matter.

Figs. 2A to 2C are views illustrating setting examples of a threshold which is set by the analyzer.

Fig. 3 is a view illustrating selection examples of pulse waves which are selected based on the threshold that is set in the above.

Fig. 4 is a view illustrating the respiratory variation which is calculated by the analyzer.

Fig. 5 is a view illustrating vital signs of the subject which are acquired by the analyzer.

Fig. 6 is a flowchart illustrating the operation of the analyzer.

Fig. 7 is a view illustrating an example of an analysis result which is displayed on a displaying section of the analyzer.

Fig. 8 is a view illustrating an example of a histogram in a modification.

Fig. 9 is a view illustrating an example of a histogram in the modification.

Fig. 10 is a view illustrating a threshold in the modification.

Fig. 11 is a view in which the respiratory variation calculated by the analyzer is compared with that calculated by a related-art calculation method.

Fig. 12 is a view illustrating a display example of a PPV trend in the case where a PCPS is used.

DETAILED DESCRIPTION OF EXEMPLARY EMBODIMENTS

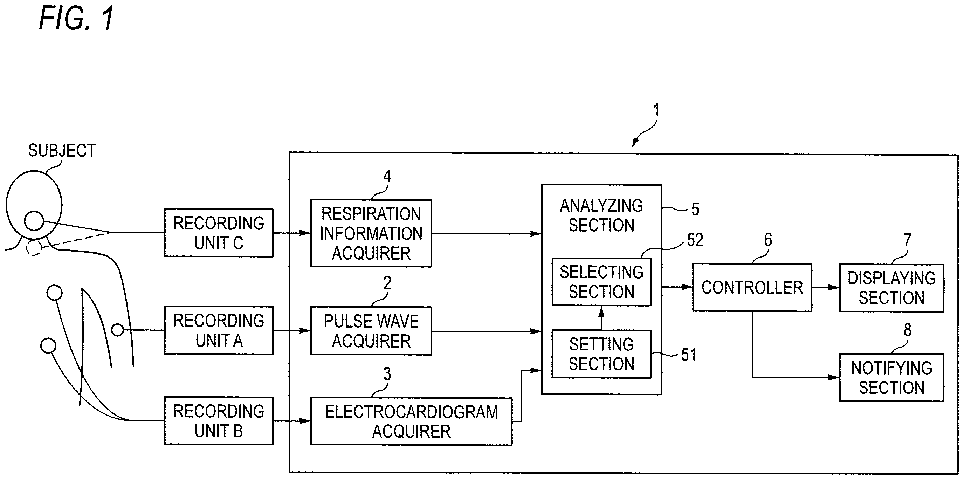

Hereinafter, an embodiment of the presently disclosed ) subject matter will be described in detail with reference to the drawings. Fig. 1 is a functional block diagram illustrating a pulse wave analyzer 1. The pulse wave analyzer 1 has a function of calculating the respiratory variation in a subject to whom an artificial respirator is attached. For example, the value indicating the respiratory variation may be selected from the PPV (Pulse Pressure Variation) indicating the change rate of the amplitude of the pulse pressure, the SPV (Systolic Pressure Variation) indicating the variation rate of the systolic pressure of the pulse pressure, the SVV (Stroke Volume Variation) indicating the change rate of the stroke volume, the PVI (Pleth Variability Index) indicating the variation rate of the pulse wave in the arterial oxygen saturation ($SpO_2$), and the like.

As illustrated in Fig. 1, the pulse wave analyzer 1 may include a pulse wave acquirer 2, an electrocardiogram acquirer 3, a respiration information acquirer 4, an analyzing section 5, a controller 6, a displaying section 7, and a notifying section 8.

The pulse wave acquirer 2 is communicably connected to a recording unit A. The pulse wave acquirer 2 acquires the arterial pressure of the subject from the recording unit A, and produces the blood pressure waveform (pulse wave) from the arterial pressure. The pulse wave is configured by a plurality of continuous unit pulse waves. A unit pulse wave means a unit of pulse wave corresponding to one heart beat. The recording unit A is configured by, for example, a catheter or the like which is to be inserted into the blood vessel of the subject. As the recording unit A, alternatively, a blood pressure measurement cuff which is to be attached to an upper arm portion, an pulse oximeter in which a probe is attached to the finger tip or the ear, or the like may be used.

The electrocardiogram acquirer 3 is communicably connected to a recording unit B. The electrocardiogram acquirer 3 acquires an electrocardiogram from the recording unit B. The recording unit B is configured by, for example, an electrocardiogram monitor having electrodes for recording a standard 3-lead electrocardiogram.

The respiration information acquirer 4 is communicably connected to a recording unit C. The respiration information acquirer 4 acquires the respiration information from the recording unit C. The recording unit C is configured by, for example, an artificial respirator which supplies and discharges (ventilates) the air through a tube attached to the subject, to assist the respiration of the subject. For example, the respiration information includes the cycle of forced ventilation which is controlled by the artificial respirator, timings when spontaneous respiration of the subject is generated, the respiratory rate in the subject, the concentration of carbon dioxide ($CO_2$ concentration) in the respiration of the subject, etc. The artificial respirator is configured so as to be able to assist also a subject in whom spontaneous respiration is generated. The artificial respirator has ventilation modes such as the A/C (Assist Control), the SIMV (Synchronized Intermittent Mandatory Ventilation), the PSV (Pressure Support Ventilation), and the CPAP (Continuous Positive Airway Pressure).

The analyzing section 5 calculates the value of the respiratory variation based on the pulse wave acquired from the subject. In the embodiment, the analyzing section 5 selects unit pulse waves from the pulse wave or the electrocardiogram, and the respiration information, and calculates the value of the respiratory variation. The analyzing section 5 may include a setting section 51 and a selecting section 52.

The setting section 51 sets a selection criterion for selecting unit pulse waves which are used in calculation of the respiratory variation. The selection criterion is, for example, a value relating to the amplitude of a unit pulse wave, and a threshold for selecting unit pulse waves based on the variation rate of the amplitudes of unit pulse waves with respect to a reference amplitude. The setting section 51 sets the threshold based on the heart rate detected from the electrocardiogram, and the respiration rate detected from the respiration information. The threshold is a value which is set for each subject based on the heart rate and respiration rate of the subject. Alternatively, the setting section 51 may set the threshold based on the pulse rate detected from the pulse wave, and the respiration rate detected from the respiration information. The threshold is a value which is set for each subject based on the pulse rate and respiration rate of the subject.

The selecting section 52 selects a pulse wave which is used in calculation of the respiratory variation, based on the predetermined threshold (selection criterion). For example, a pulse wave is selected by comparing the amplitudes of continuous unit pulse waves with one another, and eliminating a unit pulse wave (unit pulse wave which is discontinuously changed) having a variation rate which is equal to or larger than the threshold with respect to the amplitude of the immediately preceding unit pulse wave. The comparison of the amplitudes of unit pulse waves is performed, for example, within each respiration period.

The controller 6 controls the contents to be displayed on the displaying section 7, those to be notified by the notifying section 8, and the like. The displaying section 7 displays analysis information of the pulse wave which is output from the analyzing section 5. The displaying section 7 is configured by, for example, a touch-panel liquid crystal display device. The notifying section 8 notifies of an abnormality of an analysis result. The notifying section 8 is configured by, for example, a speaker.

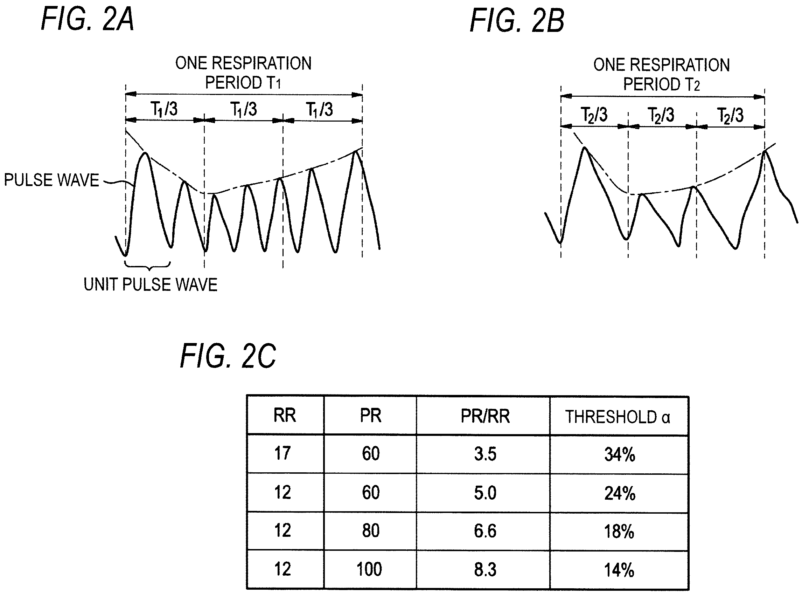

Next, an example of the threshold which is set by the setting section 51 will be described with reference to Figs. 2A to 2C.

First, the inventors attempted that, when the threshold is to be set, for example, the threshold is fixed to "threshold $\alpha_1$ = ±10%," and pulse waves are selected by eliminating unit pulse waves having a variation rate which is equal to or larger than the threshold $\alpha_1$ (±10%) with respect to the amplitude of the immediately preceding unit pulse wave. In this instance, however, there was a case where, with respect to a subject in whom the number of pulse waves contained in one respiration period, also unit pulse waves which are necessary for calculating the respiratory variation are eliminated, with the result that the accuracy of the respiratory variation is not improved. The inventors found that, when the respiratory variation is to be calculated, even a case where there are at least 3.5 beats of pulse waves in one respiration period should be included in objects. Furthermore, the inventors found that, when the measurement range of the PPV is set to 0 to 50%, there may possibly be a case where the variation rates of the maximum and minimum amplitudes of unit pulse waves are required to be a maximum of 40%. Therefore, the inventors have studied that, in comprehensive consideration of the above-described circumstances, the threshold $\alpha$ is determined in accordance with the number of pulse waves contained in one respiration period.

Fig. 2A illustrates variation (decrease/increase) of pulse waves which are due to respiratory variation in the case where 6.6 beats of unit pulse waves are included in one respiration period $T_1$. It is assumed that, when a ratio of I (Inspiration) : E (Expiration) is 1:2 in one respiration period $T_1$ (one cycle), a unit pulse wave returns in 1/3 cycle ($T_1$/3). Then, the pulse waves which are due to respiratory variation decrease or increase in 2.2 beats (6.6 beats/3) . In the case where, as described above, measurement without elimination of unit pulse waves is enabled until a pulse wave is obtained in which the variation rate of the maximum and minimum amplitudes of unit pulse waves is 40%, therefore, the variation rate which is converted for each unit pulse wave is (40%) ÷ (2.2 beats) = 18%. Therefore, unit pulse waves having a variation rate which is equal to or larger than the threshold $\alpha$ = ±18% are allowed to be eliminated.

Fig. 2B illustrates variation (decrease/increase) of pulse waves which is due to respiratory variation in the case where 3.5 beats of unit pulse waves are included in one respiration period $T_2$. Also in one respiration period $T_2$, similarly with the above case of one respiration period $T_1$, the pulse waves decrease or increase by variation of pulse waves due to respiratory variation in 1.17 beats (3.5 beats/3) included in the 1/3 cycle ($T_2$/3). Similarly with the above, when converted for each unit pulse wave, therefore, the variation rate is (40%) ÷ (1.17 beats) = 34%. Therefore, unit pulse waves having a variation rate which is equal to or larger than the threshold $\alpha$ = ±34% are allowed to be eliminated.

Fig. 2C illustrates an example of a threshold setting table which is set in a similar manner as described above. In the example, the setting section 51 calculates the number (PR/RR) of pulse waves contained in one respiration period based on the pulse rate (PR) and respiration rate (RR) in one minute, and further calculates the threshold $\alpha$ based on the number of pulse waves contained in one respiration period. The calculation expression is indicated as Threshold $\alpha$ = ±120/ (PR/RR) . As indicated in Fig. 2C, the threshold $\alpha$ is set so as to be small in the case where the number (PR/RR) of pulse waves contained in one respiration period is large, and large in the case where the number of pulse waves is small. Although the pulse rate is used in the example, an electrocardiogram (heart rate) may be used in setting of the threshold.

The threshold $\alpha$ which is calculated in each subject may be again calculated and updated at predetermined time intervals. Alternatively, the number of pulse waves contained in one respiration period may be detected in every respiration, and the threshold $\alpha$ may be calculated in every respiration.

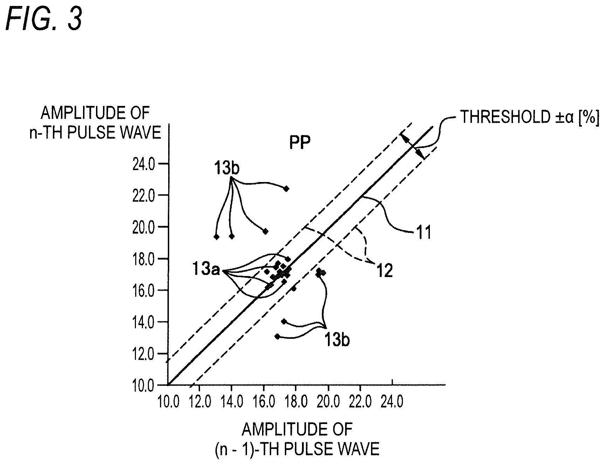

Next, a pulse wave which is selected by the selecting section 52 will be described with reference to Fig. 3.

In the graph of Fig. 3, the ordinate indicates the amplitude of the unit pulse wave to be selected, and the abscissa indicates the amplitude of the immediately preceding unit pulse wave. The solid line 11 indicates that the amplitude of the unit pulse wave (n) to be selected is equal to that of the immediately preceding unit pulse wave (n - 1), i.e., the variation rate is 0%. The broken lines 12 indicate the variation width in the case where the amplitude of the unit pulse wave (n) to be selected is varied by ± threshold $\alpha$% with respect to that of the immediately preceding unit pulse wave (n - 1) . For example, the larger variation rate, the unit pulse wave is indicated at a point 13 which is remoter from the solid line 11 in the graph. Unit pulse waves having the amplitude variation rate which is smaller than ± threshold $\alpha$% with respect to the immediately preceding unit pulse wave, such as those corresponding to points 13a in the range between the broken lines 12 in which the solid line 11 is included correspond to unit pulse waves which are to be used in calculation of the respiratory variation. Unit pulse waves having the amplitude variation rate which is equal to or larger than ± threshold $\alpha$% with respect to the immediately preceding unit pulse wave, such as those corresponding to points 13b correspond to unit pulse waves which are to be eliminated.

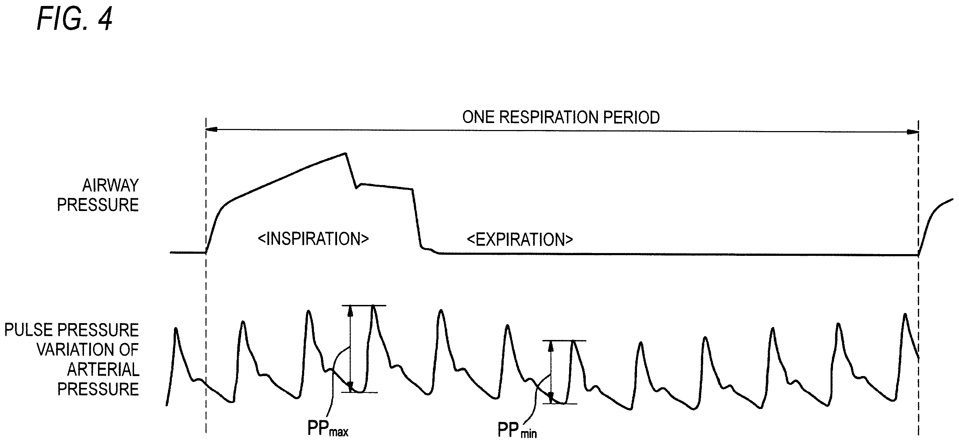

Next, the respiratory variation calculated by the analyzing section 5 will be described with reference to Fig. 4.

The respiratory variation (in the embodiment, the PPV is used) is obtained from following Expression 1:

$$PPV = (PPmax - PPmin)/((PPmax + PPmin)/2) \times 100 \, [\%]$$

$$\ldots \text{(Expression 1)}.$$

The respiratory variation which is obtained from the expression is a value which is obtained by dividing the difference between the maximum amplitude (PPmax) of the pulse wave in one period of respiration and the minimum amplitude (PPmin), by the average of the maximum amplitude of the pulse wave and the minimum amplitude, and indicates the variation rate of the amplitude levels of the pulse waves in one reciprocation period. The calculation is completed by computation on time-axis data, and obtains one variation rate per reciprocation period.

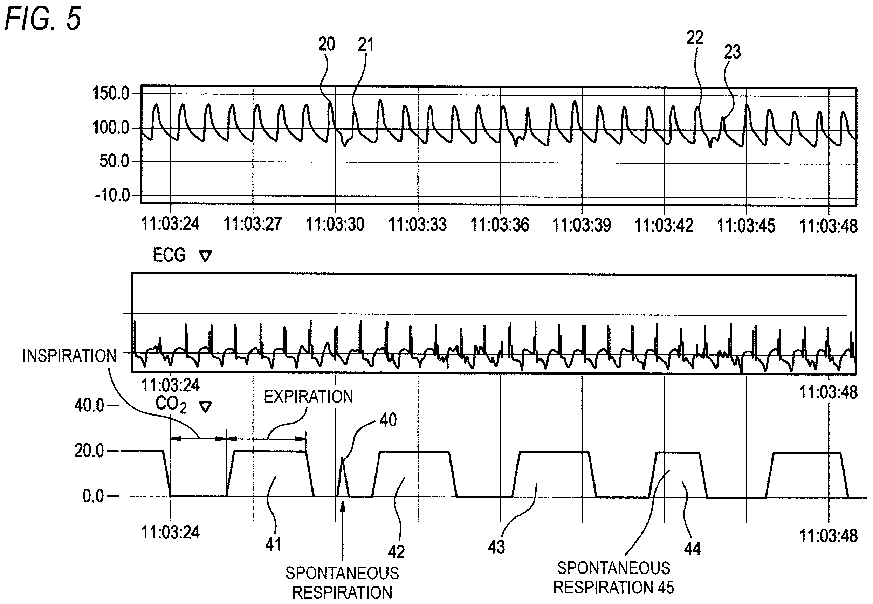

Next, vital signs acquired by the acquirers 2 to 4 will be described with reference to Fig. 5.

In the three graphs illustrated in Fig. 5, the upper graph indicates the pulse wave of the subject which is acquired by the pulse wave acquirer 2, the middle graph indicates an electrocardiogram of the subject which is acquired by the electrocardiogram acquirer 3, and the lower graph indicates the $CO_2$ concentration of the subject which is acquired by the respiration information acquirer 4. The pulse wave acquirer 2, the electrocardiogram acquirer 3, and the respiration information acquirer 4 are configured so that the timing when the pulse wave acquirer 2 acquires the pulse wave, that when the electrocardiogram acquirer 3 acquires the electrocardiogram, and that when the respiration information acquirer 4 acquires the $CO_2$ concentration are synchronized with one another.

As indicated in the graph of the $CO_2$ concentration, in the embodiment, the cycle of the forced ventilation which is controlled by the artificial respirator is set so as to be 2 seconds in inspiration, and 3 seconds in expiration. A waveform 40 is generated between expiration 41 and expiration 42. This indicates a change of the $CO_2$ concentration due to spontaneous respiration in the subject. The time period of expiration 44 is shorter than 3 seconds. This indicates that the subject generates spontaneous respiration 45 during the expiration 44.

As indicated in the graph of the pulse wave, a unit pulse wave 21 in which the variation rate is large (the amplitude is low) with respect to the amplitude of the immediately preceding unit pulse wave 20 is generated in synchronization with the generation timing of the spontaneous respiration (waveform 40) in the subject. In accordance with the generation of the spontaneous respiration 45 in the subject, a unit pulse wave 23 in which the variation rate is large (the amplitude is low) with respect to the amplitude of the immediately preceding unit pulse wave 22 is generated after the expiration 44 ends. In the embodiment, the amplitude of each of unit pulse waves which is varied by spontaneous respiration, arrhythmia, a change of the body posture, a body motion, or the like is detected, and unit pulse waves are selected based on the threshold indicating the variation rate of the amplitude.

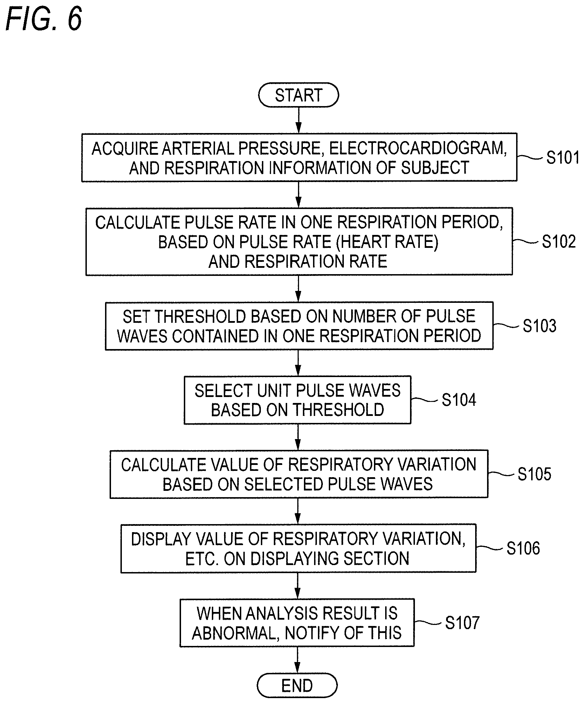

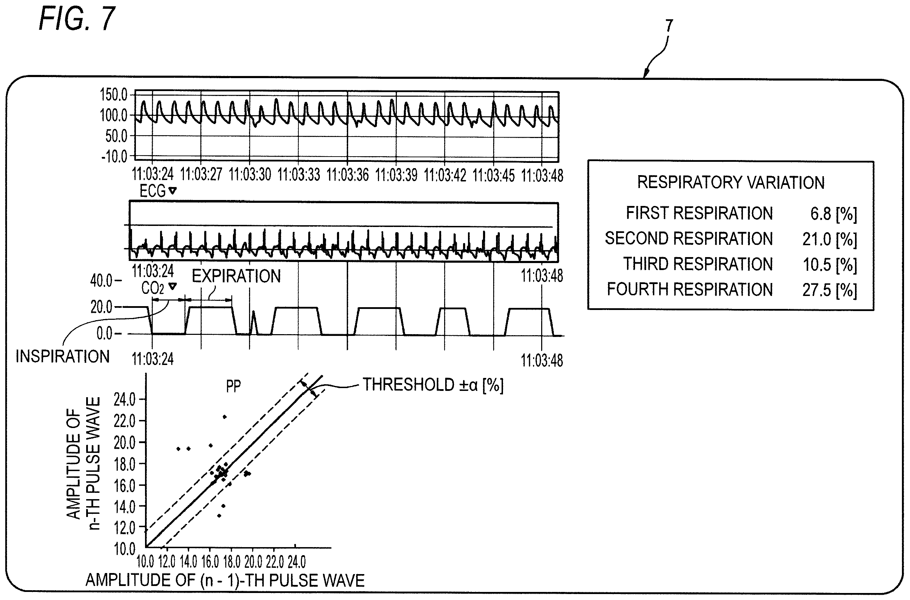

Next, the operation of the pulse wave analyzer 1 will be described with reference to Figs. 6 and 7.

As a preparatory step, the catheter (recording unit A) is inserted into the blood vessel of the subject, and electrodes of the electrocardiogram monitor (recording unit B) are attached to the body surface of the subject. A respiration information measuring unit (the recording unit C) is previously attached to the subject.

When the operation of analyzing the pulse wave is started, the pulse wave acquirer 2 acquires the arterial pressure recorded by the catheter, from the catheter, and applies signal processing on the acquired arterial pressure to take out the blood pressure waveform (pulse wave) (step S101, the upper graph of Fig. 5). Moreover, the electrocardiogram acquirer 3 acquires an electrocardiogram of the subject from the electrocardiogram monitor (step S101, the middle graph of Fig. 5). Furthermore, the respiration information acquirer 4 acquires the respiration information of the subject such as the $CO_2$ concentration (the lower graph of Fig. 5), the respiratory cycle, timings when spontaneous respiration is generated, and the respiratory rate, from the respiration information measuring unit (step S101).

The setting section 51 of the analyzing section 5 acquires pulse waves from the pulse wave acquirer 2, and detects the pulse rate of the subject based on the acquired pulse waves. The setting section 51 further acquires the respiratory rate from the respiration information acquirer 4. The setting section 51 calculates the number of pulse waves contained in one respiration period, based on, for example, the pulse rate and respiration rate in one minute (step S102).

The setting section 51 sets the threshold $\alpha$ for selecting unit pulse waves which are to be used in calculation of the respiratory variation, based on the number of pulse waves contained in one respiration period (step S103).

The selecting section 52 acquires pulse waves from the pulse wave acquirer 2, and, from the acquired pulse waves, selects pulse waves which are to be used for calculating the respiratory variation, based on $\pm$ threshold $\alpha$ which is set by the setting section 51 (step S104). Specifically, the selecting section 52 compares the amplitudes of unit pulse waves which, in the pulse waves, are continuous in one respiration period, with one another, and selects pulse waves by eliminating unit pulse waves having the amplitude variation rate which is equal to or larger than $\pm$ threshold $\alpha\%$ with respect to the amplitude of the immediately preceding unit pulse wave. The eliminated unit pulse waves are not used as unit pulse waves which are comparison objects in the comparison of the amplitude of the next unit pulse wave.

The analyzing section 5 calculates the respiratory variation in one respiration period based on the pulse waves selected by the selecting section 52 (step S105).

Then, the controller 6 causes the vital signs acquired by the acquirers 2 to 4 in step S101, the unit pulse waves which are selected by using the threshold α in step S104, the values of the respiratory variations in respective respiration cycles which are calculated in step S105, and the like to be displayed on the display device of the displaying section 7 (step S106, Fig. 7).

In the case where the value of the calculated respiratory variation exceeds a predetermined threshold, the controller 6 causes the notifying section 8 to output, for example, a warning alarm (step S107).

The respiratory variation (for example, the PPV) is expected as an important parameter for transfusion management due to insufficiency of the circulatory blood volume. In the prior art, however, the respiratory variation is obtained with many restrictions, and under the specifications that it can be measured only during stable positive-pressure respiration which is caused by an artificial respirator, and in which arrhythmia and spontaneous respiration are not generated. In, for example, an ICU (Intensive Care Unit), when the patient generates spontaneous respiration, by contrast, a ventilation mode of an artificial respirator in which the spontaneous respiration is used is frequently employed. Therefore, it is often that the value of the respiratory variation becomes unstable due to the generation of spontaneous respiration, arrhythmia, or the like.

According to the configuration of the presently disclosed subject matter, by contrast, a unit pulse wave (for example, a unit pulse wave which is discontinuously changed due to a change of the body posture, a body motion, arrhythmia, spontaneous respiration, or the like) which causes the calculation accuracy of the respiratory variation to be lowered can be eliminated based on the predetermined threshold in the calculation of the respiratory variation of each subject. The threshold can be set to a value which is suitable for the subject, in accordance with the number of pulse waves that are contained in one respiration period, the number being calculated from the respiration information (respiration rate), and pulse wave information (pulse rate) or electrocardiogram information (heart rate) of the subject. In the case where the respiration of the subject is assisted by, for example, using an artificial respirator, therefore, it is possible to accurately obtain the respiratory variation which is an index for knowing the circulatory dynamics in the subject.

Since the threshold relating to the amplitude of a pulse wave is used as the selection criterion, pulse waves which may cause the accuracy to be lowered can be efficiently eliminated in the calculation of the respiratory variation of the subject.

The threshold is set in accordance with the number of pulse waves contained in one respiration period. When the respiratory variation of the subject is to be calculated, therefore, an appropriate threshold can be set in accordance with the data number of pulse waves contained in one respiration period. Consequently, the accuracy of the respiratory variation can be further improved.

The amplitudes of unit pulse waves which are contained in one respiration period are compared with the amplitude of the immediately preceding unit pulse wave, and unit pulse waves having the amplitude variation rate which is equal to or larger than the threshold are eliminated. Therefore, unit pulse waves which are to be used in calculation of the respiratory variation can be selected more appropriately.

(Modification)

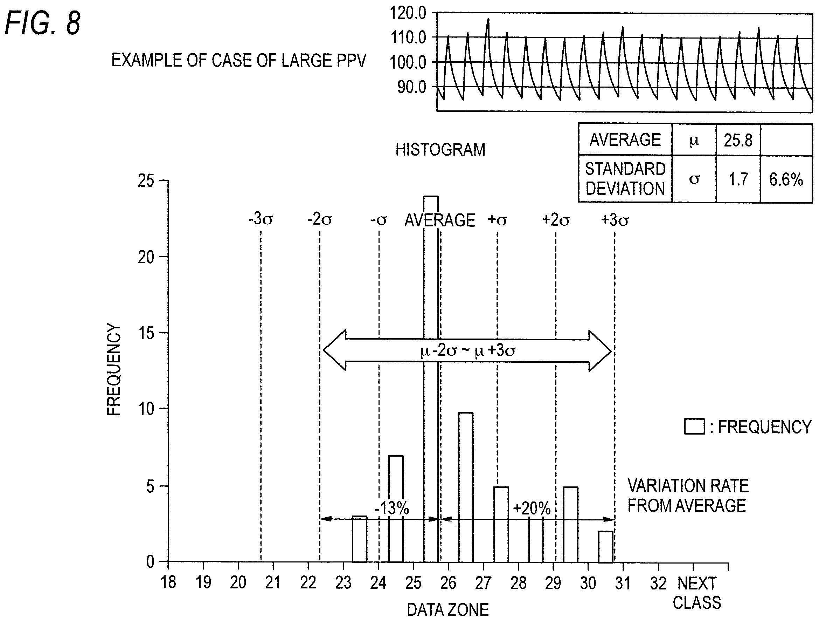

A modification of the selection of unit pulse waves in the above-described embodiment will be described with reference to Fig. 8.

Unit pulse waves which are to be used in calculation of the respiratory variation may be selected based on a variation rate with respect to the average amplitude of unit pulse waves.

In this case, the setting section calculates the average amplitude and standard deviation of unit pulse waves which are contained in pulse waves included in a predetermined time period (for example, one minute). Fig. 8 is a graph showing a histogram of the amplitudes of pulse waves in the predetermined time period. In the case where a usual I to E ratio is 1:2, a normal distribution which is bilaterally symmetric is not obtained, the frequency on the plus side with respect to the average amplitude is higher, and that on the minus side is lower. Because of the characteristics, while setting the average amplitude as the reference, a threshold is set in which the lower limit is set to be small, the upper limit is set to be larger, and the standard deviation is used (for example, the lower limit is $-2\sigma$, and the upper limit is $+3\sigma$). The threshold depends on the PPV and the standard deviation, and becomes larger as the PPV and the standard deviation are larger.

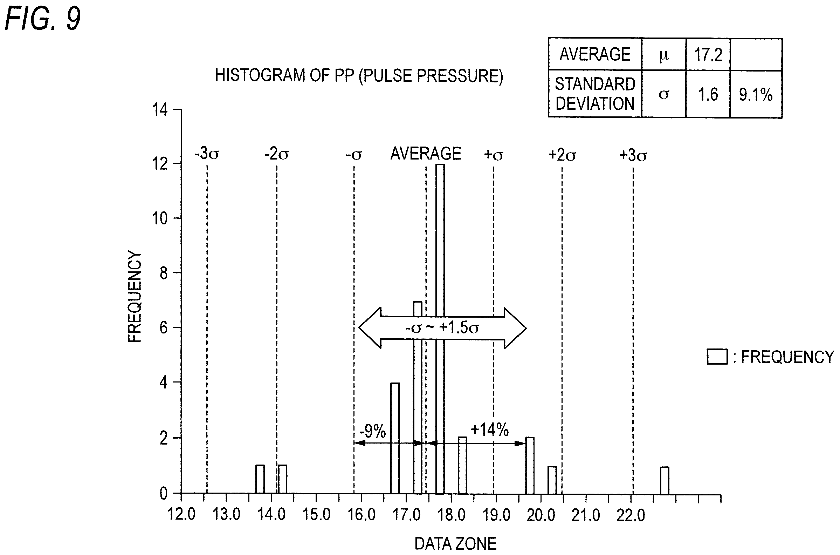

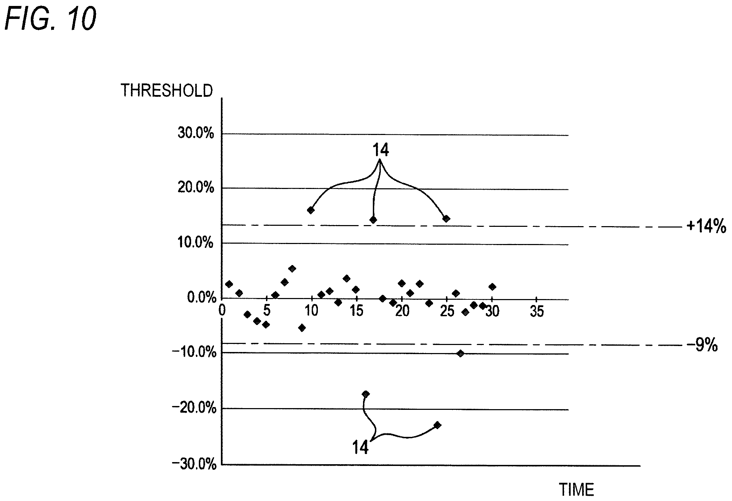

The selecting section 52 eliminates unit pulse waves which exceed the above-described threshold, among unit pulse waves in the predetermined time period (for example, one minute). Fig. 9 illustrates a histogram of the above-described example, and Fig. 10 illustrates an example in which unit pulse waves that exceed the lower limit $-\sigma$ (-9%) and upper limit $+1.5\sigma$ (+14%) of the threshold with respect to the average amplitude are eliminated. For example, unit pulse waves respectively corresponding to the points 14 correspond to unit pulse waves which are to be eliminated.

The analyzing section 5 calculates the respiratory variation in each respiration cycle based on the pulse waves selected by the selecting section 52.

According to the configuration, the preset threshold is used, unit pulse waves are eliminated based on the variation rate with respect to the average amplitude, and therefore pulse waves which are to be used in calculation of the respiratory variation can be efficiently selected.

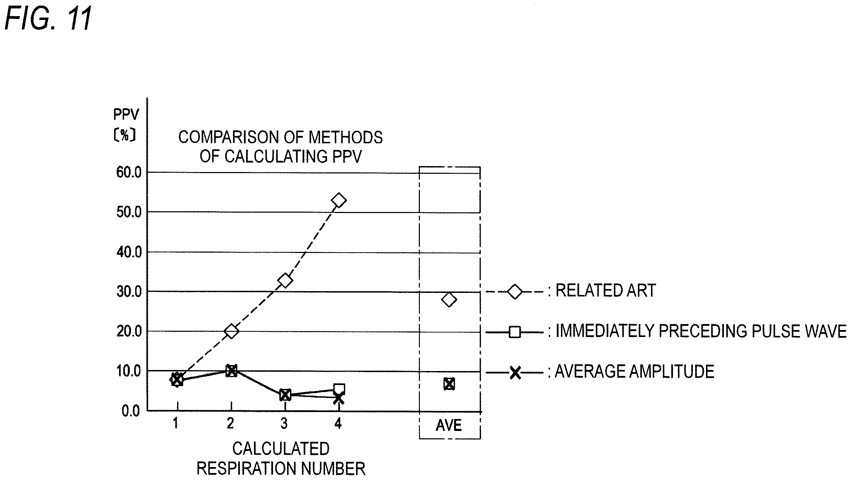

Fig. 11 is a graph in which the value of the respiratory variation calculated according to the presently disclosed subject matter is compared with that of the respiratory variation calculated by a related-art method. In Fig. 11, the abscissa indicates the sample number of one cycle of respiration in which the respiratory variation is calculated, and the ordinate indicates the value of the respiratory variation. The symbols "OPEN RHOMBUS" indicate the values of the respiratory variations that were calculated by the related-art method in which pulse waves are not selected, and all unit pulse waves are used. The symbols "OPEN SQUARE" indicate the values of the respiratory variations according to the presently disclosed subject matter which were calculated while selecting pulse waves by the amplitude variation rate with respect to the amplitude of the immediately preceding unit pulse wave. The symbols "CROSS" indicate the values of the respiratory variations according to the presently disclosed subject matter which were calculated while selecting pulse waves by the amplitude variation rate with respect to the average amplitude of unit pulse waves.

In the respiratory variation in one cycle of respiration in which a change of the body posture, a body motion, spontaneous respiration, arrhythmia, and the like did not occur in the subject (for example, Sample No. 1), the value of the respiratory variation calculated by the related-art method is approximately equal to that of the respiratory variation calculated by the presently disclosed subject matter. In the respiratory variation in one cycle of respiration in which spontaneous respiration, a body motion, spontaneous respiration, arrhythmia, or the like occurred in the subject (for example, Sample Nos. 2, 3, and 4), by contrast, the value calculated by the related-art method is largely varied, but the value calculated by the presently disclosed subject matter is approximately equal to the value of spontaneous respiration in Sample No. 1.

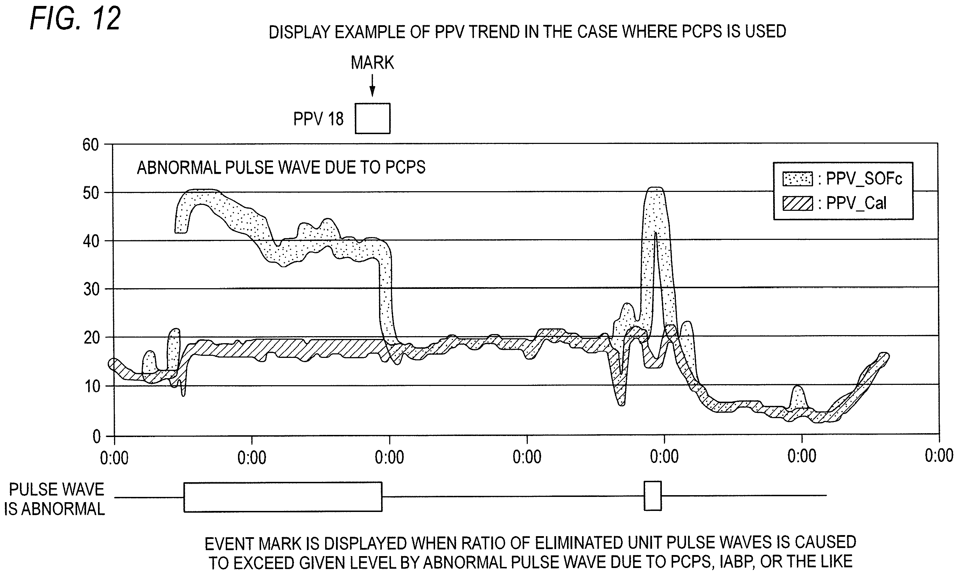

According to the method of eliminating pulse waves, the accuracy of the calculation of the respiratory variation that is an index for knowing the circulatory dynamics in the subject can be improved. In a special case, such as a case where an auxiliary circulation apparatus (an IABP, a PCPS, or the like) is used, or where an arrhythmia frequently occurs, it is highly possible that the ratio of pulse wave elimination is increased, the value obtained by the analyzer of the presently disclosed subject matter is different from that obtained by the related art, and at the same time the reliability of the PPV is lowered. In order to call attention to the fact, therefore, the analyzer has a function of, when the ratio of eliminated unit pulse waves exceeds a given level (for example, 20%), displaying a mark in the vicinity of the value of the PPV, and an event mark along the PPV trend (see Fig. 12).

The above-described embodiment is a mere example for facilitating understanding of the presently disclosed subject matter, and is not intended to limit the presently disclosed subject matter. The presently disclosed subject matter may be adequately changed or improved without departing from the spirit of the presently disclosed subject matter. It is obvious that equivalents are included within the technical scope of the presently disclosed subject matter.

Although, in the embodiment, for example, the PPV is used as the value indicating the respiratory variation as illustrated in Fig. 4, another value such as the SPV may be used.

The pulse wave analyzer of the presently disclosed subject matter includes: a pulse wave acquirer which is configured to acquire a pulse wave of a subject; an electrocardiogram acquirer which is configured to acquire an electrocardiogram of the subject; a respiration information acquirer which is configured to acquire respiration information of the subject; and an analyzing section which includes: a setting section which is configured to set a selection criterion based on the pulse wave or the electrocardiogram, and the respiration information; and a selecting section which is configured to select a pulse wave based on the selection criterion, wherein the analyzing section is configured to calculate a respiratory variation from the pulse wave which is selected by the selection section.

According to the configuration, a selection criterion which is suitable for the condition of the subject can be set by using the respiration information, and pulse wave or electrocardiogram information of the subject. When the respiratory variation which is an index for knowing the circulatory dynamics in the subject is to be calculated, it is possible to eliminate, based on the selection criterion, pulse waves which may cause the accuracy to be lowered (such as pulse waves which are caused to largely swing by spontaneous respiration, arrhythmia, a change of the body posture, a body motion, or the like). When the respiration of the subject is assisted, for example, by using an artificial respirator, therefore, the respiratory variation which is an index for knowing the circulatory dynamics in the subject can be accurately obtained.

According to the pulse wave analyzer of the presently disclosed subject matter, the accuracy of the calculation of the respiratory variation which is an index for knowing the circulatory dynamics in the subject can be improved.

**Claims**

1.  A pulse wave analyzer (1) comprising:

> a pulse wave acquirer (2) which is configured to acquire a pulse wave of a subject, said pulse wave being configured by a plurality of continuous unit pulse waves;
> an electrocardiogram acquirer (3) which is configured to acquire an electrocardiogram of the subject;

a respiration information acquirer (4) which is configured to acquire respiration information of the subject; and an analyzing section (5) which includes:

a setting section (51) which is configured to set a selection criterion based on the pulse wave or the electrocardiogram, and the respiration information; and
a selecting section (52) which is configured to select unit pulse waves of said pulse wave based on the selection criterion,

wherein
the analyzing section (5) is configured to calculate a respiratory variation from the unit pulse waves which are selected by the selection section, and
the selection criterion is a threshold relating to an amplitude of the pulse wave.

2. The pulse wave analyzer (1) according to claim 1, wherein
the setting section (51) is configured to calculate a heart rate contained in one respiration period, based on a heart rate, which is detected from the electrocardiogram, in a predetermined time period, and a respiration rate, which is detected from the respiration information, in the predetermined time period, and is configured to set the threshold based on the calculated heart rate.

3. The pulse wave analyzer (1) according to claim 2, wherein
the selecting section (52) is configured to select the unit pulse waves by comparing amplitudes of unit pulse waves which, in the pulse wave, are continuous in one respiration period, with one another, and eliminating unit pulse waves having a variation rate which is equal to or larger than the threshold with respect to an amplitude of an immediately preceding unit pulse wave.

4. The pulse wave analyzer (1) according to claim 1, wherein
the setting section (51) is configured to calculate a pulse rate contained in one respiration period, based on a pulse rate, which is detected from the pulse wave, in a predetermined time period, and a respiration rate, which is detected from the respiration information, in the predetermined time period, and is configured to set the threshold based on the calculated pulse rate.

5. The pulse wave analyzer (1) according to claim 4, wherein
the selecting section (52) is configured to select the Z unit pulse waves by comparing amplitudes of unit pulse waves which, in the pulse wave, are continuous in one respiration period, with one another, and eliminating unit pulse waves having a variation rate which is equal to or larger than the threshold with respect to an amplitude of an immediately preceding unit pulse wave.

6. The pulse wave analyzer (1) according to claim 1, wherein
the selecting section (52) is configured to select the Z unit pulse waves by calculating an average amplitude and standard deviation of unit pulse waves which are contained in the pulse wave in a predetermined time period, and eliminating unit pulse waves having a variation rate that is equal to or smaller than a lower limit, and that is equal to or larger than an upper limit, with respect to the calculated average amplitude based on the standard deviation.

7. The pulse wave analyzer (1) according to claim 1, further comprising a display section (7), wherein,
in order to indicate lowering of reliability of an analysis result, the analyzer has a function of, when a ratio of eliminated unit pulse waves is equal to or higher than a given level, displaying a mark in the vicinity of a value and a trend on the display section (7).

**Patentansprüche**

1. Pulswellenanalysator (1), umfassend:

einen Pulswellenerfasser (2), der konfiguriert ist, um eine Pulswelle eines Subjekts zu erfassen, wobei die Pulswelle durch eine Vielzahl von kontinuierlichen Einheitspulswellen konfiguriert ist;
einen Elektrokardiogrammerfasser (3), der konfiguriert ist, um ein Elektrokardiogramm des Subjekts zu erfassen;
einen Atmungsinformationserfasser (4), der konfiguriert ist, um Atmungsinformationen des Subjekts zu erfassen; und

einen Analyseabschnitt (5), der einschließt:

einen Einstellungsabschnitt (51), der konfiguriert ist, um ein Auswahlkriterium basierend auf der Pulswelle oder dem Elektrokardiogramm und den Atmungsinformationen zu konfigurieren; und
einen Auswahlabschnitt (52), der konfiguriert ist, um Einheitspulswellen der Pulswelle basierend auf dem Auswahlkriterium auszuwählen,

wobei
der Analyseabschnitt (5) konfiguriert ist, um eine Atmungsvariation aus den Einheitspulswellen zu berechnen, die durch den Auswahlabschnitt ausgewählt werden, und
das Auswahlkriterium ein Schwellenwert in Bezug auf eine Amplitude der Pulswelle ist.

2. Pulswellenanalysator (1) nach Anspruch 1, wobei
der Einstellungsabschnitt (51) konfiguriert ist, um eine in einer Atmungsperiode enthaltene Herzfrequenz zu berechnen, basierend auf einer Herzfrequenz, die von dem Elektrokardiogramm, in einer vorbestimmten Zeitperiode, detektiert wird, und einer Atmungsfrequenz, die von den Atmungsinformationen, in der vorbestimmten Zeitperiode, detektiert wird, und konfiguriert ist, um den Schwellenwert basierend auf der berechneten Herzfrequenz einzustellen.

3. Pulswellenanalysator (1) nach Anspruch 2, wobei
der Auswahlabschnitt (52) konfiguriert ist, um die Einheitspulswellen auszuwählen, indem Amplituden von Einheitspulswellen, die in der Pulswelle in einer Atmungsperiode kontinuierlich sind, miteinander verglichen werden und Einheitspulswellen mit einer Variationsrate, die gleich oder größer als der Schwellenwert in Bezug auf eine Amplitude einer unmittelbar vorausgehenden Einheitspulswelle ist, eliminiert werden.

4. Pulswellenanalysator (1) nach Anspruch 1, wobei
der Einstellungsabschnitt (51) konfiguriert ist, um eine in einer Atmungsperiode enthaltene Pulsfrequenz zu berechnen, basierend auf einer Pulsfrequenz, die von der Pulswelle, in einer vorbestimmten Zeitperiode, detektiert wird, und einer Atmungsfrequenz, die von den Atmungsinformationen, in der vorbestimmten Zeitperiode, detektiert wird, und konfiguriert ist, um den Schwellenwert basierend auf der berechneten Pulsfrequenz einzustellen.

5. Pulswellenanalysator (1) nach Anspruch 4, wobei
der Auswahlabschnitt (52) konfiguriert ist, um die Einheitspulswellen auszuwählen, indem Amplituden von Einheitspulswellen, die in der Pulswelle in einer Atmungsperiode kontinuierlich sind, miteinander verglichen werden und Einheitspulswellen mit einer Variationsrate, die gleich oder größer als der Schwellenwert in Bezug auf eine Amplitude einer unmittelbar vorausgehenden Einheitspulswelle ist, eliminiert werden.

6. Pulswellenanalysator (1) nach Anspruch 1, wobei
der Auswahlabschnitt (52) konfiguriert ist, um die Einheitspulswellen auszuwählen, indem eine Durchschnittsamplitude und eine Standardabweichung von Einheitspulswellen berechnet werden, die in der Pulswelle in einer vorbestimmten Zeitperiode enthalten sind, und indem Einheitspulswellen mit einer Variationsrate, die gleich oder kleiner als ein unterer Grenzwert ist, und die gleich oder größer als ein oberer Grenzwert ist, in Bezug auf die berechnete Durchschnittsamplitude basierend auf der Standardabweichung eliminiert werden.

7. Pulswellenanalysator (1) nach Anspruch 1, weiter umfassend einen Anzeigeabschnitt (7), wobei,
um eine Verringerung der Zuverlässigkeit eines Analyseergebnisses anzuzeigen, der Analysator eine Funktion aufweist, um, wenn ein Verhältnis von eliminierten Einheitspulswellen gleich oder höher als ein gegebenes Niveau ist, eine Markierung in der Nähe eines Wertes und einen Trend auf dem Anzeigeabschnitt (7) anzuzeigen.

**Revendications**

1. Analyseur d'ondes de pouls (1) comprenant :

un dispositif d'acquisition d'onde de pouls (2) qui est configuré pour acquérir une onde de pouls d'un sujet, ladite onde de pouls étant configurée par une pluralité d'ondes de pouls unitaires continues ;
un dispositif d'acquisition d'électrocardiogramme (3) qui est configuré pour acquérir un électrocardiogramme du sujet ;
un dispositif d'acquisition d'informations de respiration (4) qui est configuré pour acquérir des informations de respiration du sujet ; et
une section d'analyse (5) qui inclut :

une section de réglage (51) qui est configurée pour régler un critère de sélection sur la base de l'onde de pouls ou de l'électrocardiogramme, et des informations de respiration ; et
une section de sélection (52) qui est configurée pour sélectionner des ondes de pouls unitaires de ladite onde de pouls sur la base du critère de sélection,

dans lequel
la section d'analyse (5) est configurée pour calculer une variation respiratoire à partir des ondes de pouls unitaires qui sont sélectionnées par la section de sélection, et
le critère de sélection est un seuil relatif à une amplitude de l'onde de pouls.

2. Analyseur d'ondes de pouls (1) selon la revendication 1, dans lequel
la section de réglage (51) est configurée pour calculer une fréquence cardiaque contenue dans une période de respiration, sur la base d'une fréquence cardiaque, qui est détectée à partir de l'électrocardiogramme, dans une période de temps prédéterminée, et d'une fréquence de respiration, qui est détectée à partir des informations de respiration, dans la période de temps prédéterminée, et est configurée pour régler le seuil sur la base de la fréquence cardiaque calculée.

3. Analyseur d'ondes de pouls (1) selon la revendication 2, dans lequel
la section de sélection (52) est configurée pour sélectionner les ondes de pouls unitaires en comparant des amplitudes d'ondes de pouls unitaires qui, dans l'onde de pouls, sont continues dans une période de respiration, les unes par rapport aux autres, et en éliminant des ondes de pouls unitaires présentant un taux de variation qui est égal ou supérieur au seuil par rapport à une amplitude d'une onde de pouls unitaire immédiatement précédente.

4. Analyseur d'ondes de pouls (1) selon la revendication 1, dans lequel
la section de réglage (51) est configurée pour calculer une fréquence de pouls contenue dans une période de respiration, sur la base d'une fréquence de pouls, qui est détectée à partir de l'onde de pouls, dans une période de temps prédéterminée, et d'une fréquence de respiration, qui est détectée à partir des informations de respiration, dans la période de temps prédéterminée, et est configurée pour régler le seuil sur la base de la fréquence de pouls calculée.

5. Analyseur d'ondes de pouls (1) selon la revendication 4, dans lequel
la section de sélection (52) est configurée pour sélectionner les ondes de pouls unitaires en comparant des amplitudes d'ondes de pouls unitaires qui, dans l'onde de pouls, sont continues dans une période de respiration, les unes par rapport aux autres, et en éliminant des ondes de pouls unitaires présentant un taux de variation qui est égal ou supérieur au seuil par rapport à une amplitude d'une onde de pouls unitaire immédiatement précédente.

6. Analyseur d'ondes de pouls (1) selon la revendication 1, dans lequel
la section de sélection (52) est configurée pour sélectionner les ondes de pouls unitaires en calculant une amplitude moyenne et un écart type d'ondes de pouls unitaires qui sont contenues dans l'onde de pouls dans une période de temps prédéterminée, et en éliminant des ondes de pouls unitaires présentant un taux de variation qui est égal ou inférieur à une limite inférieure, et qui est égal ou supérieur à une limite supérieure, par rapport à l'amplitude moyenne calculée sur la base de l'écart type.

7. Analyseur d'ondes de pouls (1) selon la revendication 1, comprenant en outre une section d'affichage (7), dans lequel, afin d'indiquer une baisse de fiabilité d'un résultat d'analyse, l'analyseur présente une fonction consistant à, lorsqu'un rapport d'ondes de pouls unitaires éliminées est égal ou supérieur à un niveau donné, afficher une marque à proximité d'une valeur et d'une tendance sur la section d'affichage (7).

FIG. 1

FIG. 2A

FIG. 2B

FIG. 2C

| RR | PR | PR/RR | THRESHOLD α |
|----|----|-------|-------------|
| 17 | 60 | 3.5 | 34% |
| 12 | 60 | 5.0 | 24% |
| 12 | 80 | 6.6 | 18% |
| 12 | 100 | 8.3 | 14% |

*FIG. 3*

## FIG. 4

ONE RESPIRATION PERIOD

AIRWAY
PRESSURE

<INSPIRATION>            <EXPIRATION>

PULSE PRESSURE
VARIATION OF
ARTERIAL
PRESSURE

$PP_{max}$            $PP_{min}$

EP 3 158 927 B1

FIG. 5

## FIG. 6

START

ACQUIRE ARTERIAL PRESSURE, ELECTROCARDIOGRAM, AND RESPIRATION INFORMATION OF SUBJECT ⟋ S101

CALCULATE PULSE RATE IN ONE RESPIRATION PERIOD, BASED ON PULSE RATE (HEART RATE) AND RESPIRATION RATE ⟋ S102

SET THRESHOLD BASED ON NUMBER OF PULSE WAVES CONTAINED IN ONE RESPIRATION PERIOD ⟋ S103

SELECT UNIT PULSE WAVES BASED ON THRESHOLD ⟋ S104

CALCULATE VALUE OF RESPIRATORY VARIATION BASED ON SELECTED PULSE WAVES ⟋ S105

DISPLAY VALUE OF RESPIRATORY VARIATION, ETC. ON DISPLAYING SECTION ⟋ S106

WHEN ANALYSIS RESULT IS ABNORMAL, NOTIFY OF THIS ⟋ S107

END

FIG. 7

FIG. 8

FIG. 9

HISTOGRAM OF PP (PULSE PRESSURE)

| AVERAGE | μ | 17.2 | |
|---|---|---|---|
| STANDARD DEVIATION | σ | 1.6 | 9.1% |

FIG. 10

EP 3 158 927 B1

EP 3 158 927 B1

FIG. 11

## FIG. 12

DISPLAY EXAMPLE OF PPV TREND IN THE CASE WHERE PCPS IS USED

PPV 18 [ MARK ]

ABNORMAL PULSE WAVE DUE TO PCPS

Legend:
- ▨ : PPV_SOFc
- ▧ : PPV_Cal

PULSE WAVE IS ABNORMAL

EVENT MARK IS DISPLAYED WHEN RATIO OF ELIMINATED UNIT PULSE WAVES IS CAUSED TO EXCEED GIVEN LEVEL BY ABNORMAL PULSE WAVE DUE TO PCPS, IABP, OR THE LIKE

EP 3 158 927 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011511686 T
- US 2009156945 A1
- US 2009048527 A1
- EP 2263528 A1